# FASCICULE DE BREVET EUROPEEN

(11) **EP 2 988 765 B1**
(45) Date de publication et mention de la délivrance du brevet: **03.07.2019**
(21) Numéro de dépôt: 14729387.2
(22) Date de dépôt: 24.04.2014
(51) Int. Cl.: A61K 36/55, A61K 8/73, A61K 8/97, A61Q 19/08, A61K 31/702, A61P 17/02

(54) **PROCÉDÉ D'OBTENTION D'UN MÉLANGE D'OLIGOSACCHARIDES NEUTRES EXTRAITS DE GRAINES DE LIN**
VERFAHREN ZUR HERSTELLUNG EINER MISCHUNG VON NEUTRALEN OLIGOSACCHARIDEN AUS FLACHSSAMEN
METHOD FOR PRODUCING A MIXTURE OF NEUTRAL OLIGOSACCHARIDES EXTRACTED FROM FLAXSEED

(30) Priorité: 24.04.2013 FR 1353718
(43) Date de publication de la demande: 02.03.2016
(73) Titulaire: Université De Reims Champagne-Ardenne, 51100 Reims (FR); Centre Valorisation Glucides Prod Nat, 80480 Dury (FR); Universite de Rouen, 76130 Mont-Saint-Aignan (FR); Vandeputte Oleochemicals, 7700 Mouscron (BE); Centre National de la Recherche Scientifique, 75794 Paris Cedex 16 (FR)
(72) Inventeur: MAQUART, François, F-51100 Reims (FR); BELLON, Georges, F-51100 Reims (FR); MARCHAL, Claire, F-67200 STRASBOURG (FR); DUCATEL, Hélène, F-60120 Beauvoir (FR); DUPUIS, Olivier, F-62270 Frevent (FR); PICTON, Luc, F-76530 Grand Couronne (FR); LECERF, Didier, F-76750 Bosc Roger Sur Buchy (FR); FORBICE, Renauld, F-83300 Draguignan (FR)
(74) Mandataire: Rhein, Alain
(86) Numéro de dépôt international: PCT/FR2014/050994
(87) Numéro de publication internationale: WO 2014/174220

(56) Documents cités:
- WO-A1-93/16707
- WO-A2-2008/043944
- KARINE GUILLOUX ET AL: "Production of Arabinoxylan-oligosaccharides from Flaxseed ( Linum usitatissimum )", JOURNAL OF AGRICULTURAL AND FOOD CHEMISTRY, vol. 57, no. 23, 9 décembre 2009 (2009-12-09), pages 11308-11313, XP055095419, ISSN: 0021-8561, DOI: 10.1021/jf902212z
- WOJTASIK W ET AL: "The significance of flax fibre pectin in the extracellular matrix remodelling of wound healing process", FEBS JOURNAL, BLACKWELL PUBLISHING, LONDON, GB, vol. 279, no. Suppl. 1, 1 septembre 2012 (2012-09-01), page 72, XP009175407, ISSN: 1742-464X, DOI: 10.1111/J.1742-4658.2010.08705.X [extrait le 2012-08-30]

## Description

La présente invention concerne les oligosaccharides, notamment issus des végétaux, et plus particulièrement l'utilisation de ces oligosaccharides dans le domaine cosmétique ou dermatologique.

L'invention concerne plus particulièrement une utilisation cosmétique ou dermatologique des oligosaccharides neutres issus de graines de lin et un procédé d'obtention de ces oligosaccharides par exemple après hydrolyse et fractionnement d'une solution de mucilage.

En particulier, les oligosaccharides neutres issus de graines de lin peuvent notamment être utilisés pour favoriser la réparation tissulaire cutanée et également pour prévenir des effets du vieillissement cutané.

La réparation cutanée est constituée par un ensemble de processus mis en place dans l'optique de réparer un dommage ou une lésion subis par la peau et de reconstituer un tissu proche du tissu original lésé.

Le vieillissement cutané, quant à lui, est responsable de la modification des structures anatomiques et histologiques et de l'altération du fonctionnement des cellules.

Le vieillissement cutané résulte de différents facteurs, notamment une altération cellulaire, due par exemple au stress oxydatif, mais également des facteurs externes, tels que la pollution, la consommation de tabac ou d'alcool, une exposition trop importante aux rayons solaires, etc.

Le vieillissement cutané se traduit par l'apparition de rides et de tâches pigmentaires, généralement de couleur brune et par une diminution du tonus de la peau.

L'état de la technique propose un certain nombre de stratégies destinées à lutter contre les manifestations visibles du vieillissement cutané. A ce titre, on peut notamment citer les techniques suivantes :
- le traitement au laser pour corriger les défauts tels que les tâches brunes et les rides fines ;
- les injections de toxine botulinique, d'acide hyaluronique ou encore de collagène ;
- le peeling, qui consiste en un retrait d'une couche importante de l'épiderme ;
- le lifting ;
- etc.

Cependant, les techniques existant actuellement présentent un certains nombres d'effets secondaires indésirables et ne permettent pas toujours d'obtenir des résultats satisfaisants.

En conséquence, depuis quelques années, d'autres stratégies sont mises en place par les laboratoires dans le but de lutter de manière plus efficace et de façon plus naturelle contre les effets du vieillissement cutané et pour tenter de ralentir ce processus. Il convient également de trouver des solutions pour favoriser la réparation tissulaire cutanée en cas de lésions.

En particulier, les recherches sur l'utilisation d'oligosaccharides dans les compositions à but cosmétique pour diminuer les signes du vieillissement cutané se sont développées ces dernières années. En effet, les molécules à base de sucres présentent un intérêt particulier, notamment du fait de leurs propriétés hydratantes.

Ainsi, certaines demandes de brevet sont relatives à des compositions cosmétiques comportant des oligosaccharides ; en particulier, la demande de brevet WO 99/24009 décrit notamment l'utilisation des oligosaccharides contenant du xylose pour augmenter la synthèse de certains composés, les protéoglycannes et les glycosaminoglycannes. Cependant, dans ce document, le xylose utilisé est un produit commercial, et non un produit naturel issu du végétal.

Le document de brevet US 2007/0293433 est relatif à une composition anti-âge comportant une pluralité d'oligosaccharides obtenue par hydrolyse enzymatique de la pectine, qui consiste également en un produit commercial.

On connait encore, de la demande de brevet US2004/0097464, une composition comprenant un mélange d'oligosaccharides, notamment du fucose, celui-ci étant obtenu par une hydrolyse effectuée par un micro-organisme. Le mélange d'oligosaccharides décrit ici nécessite la mise en oeuvre d'un procédé compliqué et comportant un grand nombre d'étapes, dont certaines nécessitent l'action de micro-organismes pathogènes qui doivent nécessairement être éliminés du mélange destiné à une application cosmétique.

De plus, les compositions décrites dans les documents susmentionnés ne présentent pas une action suffisamment efficace, notamment dans la stimulation de la réparation cutanée pour laquelle il existe peu de solutions dans l'état de la technique.

Il est également connu d'utiliser des principes actifs issus de végétaux, notamment de lin (*Linum usitatissimum*), plante appartenant à la famille des Linaceae, pour une utilisation dans le but de lutter contre les manifestations de l'âge.

On connait ainsi, par la demande internationale de brevet WO2008/043944 l'utilisation de principes actifs issus de lin pour la préparation d'une composition cosmétique pour lutter contre le vieillissement cutané, ainsi qu'un procédé pour les obtenir.

Cependant, le principe actif, obtenu par le procédé décrit dans cette demande de brevet, comporte un certain nombre de composants, et en majorité des protéines de masse molaire inférieure à 5000 Da en grande concentration, des sucres monomères, des acides uroniques, des carbohydrates, etc.

En conséquence, cette variété de composants ne permet pas une action ciblée et spécifique et notamment, une telle disparité de constituants ne permet pas de favoriser efficacement la réparation cutanée en cas de dommages subis par la peau.

Dans le cadre de la présente invention, les demanderesses ont découvert que certains oligosaccharides particuliers, issus de la graine de lin, permettent une stimulation efficace des phénomènes mis en oeuvre notamment dans les processus de réparation tissulaire cutanée, et ont mis au point un procédé pour l'extraction de ces oligosaccharides.

Plus particulièrement, il s'agit des oligosaccharides neutres ayant une masse molaire élevée, ces derniers étant obtenus suite à un fractionnement par ultrafiltration sur seuil de coupure compris d'une part entre 5 000 et 15 000 Da et d'autre part entre 15 000 et 50 000 Da.

Ainsi, la présente invention concerne un procédé d'obtention d'un mélange d'oligosaccharides neutres extraits de graines de lin, lesdits oligosaccharides présentant des masses molaires élevées, ledit procédé comportant les étapes suivantes :
- on effectue une hydrolyse à pH acide d'une solution de mucilage de lin, cette dernière étant obtenue par extraction de graines de lin dans un solvant, ladite hydrolyse étant effectué à un pH de 2, à une température de l'ordre de 80°C, pendant une durée de 24h ;
- on neutralise ladite solution par ajout d'une base en quantité adéquate ;
- on effectue une première ultrafiltration de la solution au travers d'une membrane de porosité 50 000 Da, de sorte à obtenir un premier rétentat et un premier perméat ;
- on effectue une seconde ultrafiltration dudit premier perméat au travers d'une membrane de porosité 15 000 Da, de sorte à obtenir un second rétentat et un second perméat ;
- on effectue une troisième ultrafiltration dudit second perméat au travers d'une membrane de porosité 5 000 Da de sorte à obtenir un troisième rétentat et un troisième perméat ;
- on mélange le second rétentat et le troisième rétentat pour obtenir ledit mélange d'oligosaccharides, ledit second rétentat comportant des oligosaccharides ayant des masses molaires issues du fractionnement par ultrafiltration sur seuil de coupure compris entre 15 000 et 50 000 Da, et ledit troisième rétentat comportant les oligosaccharides ayant des masses molaires issues du fractionnement par ultrafiltration sur seuil de coupure entre 5 000 et 15 000 Da.

Selon d'autres caractéristiques particulières du procédé :
- l'extraction de graines de lin est préférentiellement réalisée dans un solvant aqueux ;
- de préférence, l'on neutralise ladite solution de mucilage par ajout, en quantité adéquate, d'une base forte choisie dans le groupe comprenant au moins l'hydroxyde de baryum et l'hydroxyde de sodium.

La présente invention concerne également un mélange d'oligosaccharides neutres extraits de graines de lin obtenu par la mise en oeuvre du procédé selon l'invention, ledit mélange comportant des oligosaccharides ayant des masses molaires issues d'un fractionnement par ultrafiltration sur seuil de coupure compris d'une part entre 15 000 et 50 000 Da et d'autre part entre 5 000 et 15 000 Da.

De façon particulièrement intéressante, ledit mélange comporte des oligosaccharides dont la chaîne comprend au moins du fucose et/ou de l'arabinose et/ou du galactose et/ou du glucose et/ou du xylose.

Avantageusement, ledit mélange comporte un faible taux d'oligosaccharides dont la chaîne comprend du rhamnose et un faible taux d'acides uroniques.

Le mélange d'oligosaccharides neutres extraits de graines de lin peut avantageusement être utilisé à des fins cosmétiques pour lutter contre les effets du vieillissement cutané.

Préférentiellement, l'invention concerne l'utilisation dudit mélange pour stimuler :
- la prolifération des fibroblastes ;
- le chimiotactisme des fibroblastes ;
- la migration cellulaire des fibroblastes ;
- la synthèse de collagène de type III et/ou de type IV par les fibroblastes ;
L'utilisation du mélange d'oligosaccharides neutres extraits de graines de lin permet également avantageusement de stimuler la synthèse de lumicane et d'inhiber la synthèse de décorine par les fibroblastes.

Ledit mélange peut également être utilisé pour induire la différenciation des kératinocytes.

La présente invention concerne également une composition dermatologique ou cosmétique comprenant un mélange d'oligosaccharides neutres selon l'invention et au moins un véhicule cosmétiquement ou dermatologiquement acceptable.

Avantageusement, dans la composition, la concentration en oligosaccharides neutres est comprise entre 0,1 et 5mg/mL.

Selon un mode de réalisation particulier, le mélange d'oligosaccharides neutres extraits de graines de lin selon l'invention peut également être utilisé comme médicament, en particulier pour favoriser la cicatrisation des tissus, notamment dans les ulcères chroniques ou après une intervention chirurgicale.

Le mélange d'oligosaccharides neutres extraits de graines de lin peut encore être utilisé pour favoriser la réparation tissulaire cutanée, notamment favoriser la cicatrisation d'une plaie.

D'autres caractéristiques et avantages de l'invention ressortiront de la description détaillée qui va suivre des modes de réalisation non limitatifs de l'invention, en référence aux figures annexées dans lesquelles :
- la figure 1 consiste en un tableau récapitulant les caractéristiques des échantillons E1 à E7 obtenus à partir d'un mucilage de graines de lin ; les fractions E4 et E6, qui présentent un intérêt particulier, sont entourées dans le tableau ;
- les figures 2A, B et C qui illustrent, par des histogrammes dans lesquels le nombre de cellules vivantes est apprécié par colorimétrie, l'absence de cytotoxicité des oligosaccharides neutres de graines de lin sur les fibroblastes mis en présence de différentes concentrations en oligosaccharides ;
- la figure 3 représente l'effet des oligosaccharides neutres de graines de lin sur la prolifération des fibroblastes après différents temps d'incubation (24, 48 et 72h) ;
- la figure 4 illustre par des histogrammes le nombre de cellules ayant migré en fonction de leur exposition ou non à des oligosaccharides neutres issus de graines de lin ;
- la figure 5 illustre également la migration des fibroblastes en fonction de concentrations différentes en oligosaccharides neutres issus de graines de lin ;
- la figure 6 est une représentation graphique de la trajectoire des fibroblastes en présence ou en absence de fractions oligosaccharidiques ;
- les figures 7A et 7B représentent graphiquement, sous forme d'histogrammes, la synthèse de collagène de type III en présence d'oligosaccharides pendant une durée de 24 (figure 7A) ou 48h (figure 7B) ;
- la figure 8 correspond à une photographie de membranes de western blot révélant l'expression du collagène de type IV dans les fibroblastes en présence ou en absence d'oligosaccharides de graines de lin.
- la figure 9 correspond à une photographie de gels d'agarose révélant l'expression des gènes des protéoglycannes décorine et lumicanne dans les fibroblastes en présence ou en absence d'oligosaccharides de graines de lin ; le gène de la GAPDH est utilisé en tant que contrôle.

La présente invention concerne un procédé pour l'obtention d'un mélange d'oligosaccharides neutres provenant de graines de lin, notamment de la variété *Linum usitatissimum.* Ledit mélange est avantageusement obtenu par hydrolyse contrôlée puis fractionnement à partir d'une solution de mucilage de graines de lin, cette dernière étant quant à elle obtenue par extraction desdites graines dans un solvant.

Dans la suite de la description, on entend par « oligosaccharide » tout oligomère ou polymère osidique issu de l'hydrolyse acide à chaud du mucilage et dont la masse molaire permet le passage au travers de l'ultrafiltration à 50 000 Da.

On entend par « oligosaccharide neutre » un oligosaccharide qui ne comporte pas de charge et pas de résidus N-acétyl.

On entend par « mucilage » une substance végétale constituée de polymères osidiques, en particulier des polysaccharides, enveloppant notamment les graines de lin.

On entend par « solution de mucilage » la solution obtenue après trempage des graines de lin dans un solvant, notamment aqueux, par exemple l'eau, et constituée en grande partie par des polymères osidiques.

Avantageusement, le procédé selon l'invention permet l'obtention d'un mélange d'oligosaccharides neutres issus de graines de lin comportant des oligosaccharides ayant des masses molaires issues d'un fractionnement par ultrafiltration sur seuil de coupure compris d'une part entre 15 000 et 50 000 Da et d'autre part entre 5 000 et 15 000 Da.

L'invention est également relative à un mélange d'oligosaccharides neutres issus de graines de lin obtenu par la mise en oeuvre du procédé.

La présente invention concerne encore une composition, notamment présentant un usage cosmétique ou dermatologique, et comportant un mélange d'oligosaccharides neutres extraits de graines de lin obtenu par le présent procédé. Outre ces oligosaccharides, ladite composition comporte également avantageusement au moins un véhicule cosmétiquement ou dermatologiquement acceptable.

La composition selon l'invention peut se présenter indifféremment sous la forme d'une crème, d'un gel, d'une lotion, d'un sérum, d'une mousse ou encore d'une pommade.

Ladite composition est plus particulièrement destinée à être appliquée sur la peau ou encore au niveau des phanères.

La peau est un organe complexe recouvrant intégralement le corps. Elle assure plusieurs fonctions nécessaires à la survie de l'organisme, notamment la protection contre toute agression extérieure, que celle-ci soit physique, chimique ou encore biologique.

La peau est constituée de trois couches principales : la partie superficielle, l'épiderme, suivie d'une couche plus épaisse, le derme, et de la couche la plus profonde, l'hypoderme, au sein desquelles les cellules interagissent ensemble afin d'assurer les fonctions de la peau.

L'épiderme est la couche en contact direct avec l'environnement extérieur. Il protège l'organisme en empêchant l'entrée des agents pathogènes et en maintenant l'eau et les nutriments à l'intérieur. Il a une épaisseur moyenne de 100 µm, mais celle-ci peut varier considérablement selon la région du corps et le niveau de kératinisation.

La principale cellule de l'épiderme est le kératinocyte. Il possède de nombreux rôles notamment dans les réponses cutanées inflammatoires et immunitaires, constituant ainsi une barrière protectrice.

L'épiderme est séparé du derme par une membrane appelée membrane basale, dont le constituant principal est le collagène de type IV.

Le derme est la couche qui donne la souplesse et la résistance à la peau. Son épaisseur varie considérablement selon la localisation anatomique.

Le derme est composé principalement de tissus conjonctifs qui le rendent compressible et élastique. Il constitue un support pour les différentes annexes cutanées, telles que les vaisseaux sanguins, les poils, les terminaisons nerveuses et les glandes sébacées et sudoripares.

Ces annexes sont entourées de fibres majoritairement constituées de collagène de type I et III, lesquelles assurent souplesse et flexibilité au derme.

Le collagène est l'un des principaux constituants de la matrice extracellulaire. C'est une protéine qui est notamment synthétisée lors des processus de réparation tissulaire. Cependant, la synthèse de collagène est de moins en moins importante en fonction de l'âge.

Les fibroblastes sont les cellules majoritaires du derme ; ils synthétisent tous les types de fibres, notamment les fibres de collagène, ainsi que d'autres constituants de la membrane basale.

Les fibroblastes dermiques sont une composante essentielle de la peau : ils produisent et organisent la matrice extracellulaire du derme et communiquent avec d'autres types cellulaires, jouant un rôle crucial dans la régulation de la physiologie de la peau.

Au cours du temps, la peau peut subir de nombreuses agressions internes comme externes. Lorsque la peau subit un dommage ou une lésion, un ensemble de processus se met en place pour réparer ce dommage et reconstituer un tissu aussi proche que possible du tissu original.

En particulier, le vieillissement est un facteur majeur d'altérations cutanées. Il modifie les structures anatomiques et histologiques et altère le fonctionnement des cellules. La peau subit ainsi de profonds remaniements.

Le vieillissement cutané découle de la conjonction de différents facteurs, complexes et intimement liés.

En particulier, le vieillissement cutané résulte de nombreuses altérations cellulaires, telle qu'une diminution des télomères, un stress oxydatif, une dégradation des systèmes de réparation de l'ADN, auxquelles s'ajoutent des facteurs externes tels que l'exposition solaire, la pollution, les agressions climatiques, mais aussi la consommation d'alcool ou de tabac ou encore l'alimentation.

Le vieillissement de l'épiderme se manifeste principalement par la diminution de son épaisseur. Cette atrophie est la conséquence, d'une part, de l'accumulation de kératinocytes sénescents et, d'autre part, de la perte progressive des invaginations caractéristiques de l'épiderme.

Les kératinocytes basaux montrent une disparité de taille et de forme, suggérant des altérations morphologiques, ainsi qu'une diminution de leur capacité de prolifération. De plus, la capacité de régénération de l'épiderme, suite à un traumatisme, diminue avec l'âge.

Par ailleurs les propriétés d'adhérence de l'épiderme déclinent en raison d'une diminution de l'expression de l'intégrine bêta-1 impliquée dans l'adhésion des kératinocytes basaux à la lame basale sous-jacente. Le collagène IV de la lame basale, qui sert à la cohésion de la jonction dermo-épidermique et à l'adhésion des kératinocytes, diminue également au cours du vieillissement

Enfin, le vieillissement entraîne une diminution du nombre de mélanocytes et de cellules de Langerhans, affectant la capacité de protection épidermique contre les agressions.

Au cours du vieillissement cutané, le derme est également le siège d'une profonde désorganisation avec une matrice extracellulaire qui apparait dense et peu vascularisée. Son atrophie est principalement due à une réduction du nombre et de la taille des fibroblastes mais surtout de leur capacité de synthèse.

En particulier, les fibrilles de collagène prennent un aspect granuleux et les fibres deviennent plus compactes. Elles se dissocient et tendent à s'orienter parallèlement à la surface du derme. Les altérations des fibres de collagène ainsi que du matériel élastique, le changement du contenu en protéoglycannes et l'apparition de fibroblastes à l'état quiescent sans contact direct avec les faisceaux de collagène représentent les principales caractéristiques d'un derme vieillissant.

L'ensemble de ces altérations histologiques et biochimiques a des conséquences fonctionnelles cruciales sur les propriétés de la peau, engendrant des réponses moins bien adaptées aux différentes agressions extérieures.

La réparation tissulaire est un exemple de réponse naturelle lorsqu'un tissu est endommagé par une agression extérieure. Ce processus se fait systématiquement et se déroule en trois phases : inflammation, prolifération et maturation.

Les premiers événements lors d'une réparation tissulaire sont caractérisés par une phase inflammatoire, une réponse vasculaire et cellulaire. Parmi ceux-ci, on observe l'arrivée de nombreuses cellules inflammatoires, endothéliales et vasculaires. Ceci se fait *via* la production de facteurs qui ont des propriétés chimio-attractantes.

Durant la phase de prolifération, la formation d'un tissu granuleux est l'événement-clé. Les cellules inflammatoires, les fibroblastes, les molécules de la matrice extracellulaire (fibronectine, collagène, glycosaminoglycannes et protéoglycannes) forment le tissu de granulation. Celui-ci se forme 3 à 5 jours suivant l'agression et survient après la phase inflammatoire.

Lorsque l'épithélium a été lésé, les cellules épidermiques aux abords de la blessure se multiplient et migrent pour recouvrir le tissu sain. Quand le processus est complet, les cellules épidermiques reprennent leurs formes et leurs rôles initiaux. Durant cette phase, une fibroplasie se met en place : les fibroblastes jouent un rôle très important dans cette étape. Ils sont responsables de la production de collagène (et plus particulièrement du collagène de type III), d'élastine, de fibronectine et de glycosaminoglycanne. Par ailleurs, les fibroblastes migrent et prolifèrent.

La troisième phase est la phase de maturation et de remodelage où se termine la réparation dermique et épidermique.

Pendant cette phase, il se produit une maturation des fibres et une apoptose cellulaire qui conduit à la formation d'un tissu proche du tissu initial. Le remodelage se fait notamment au niveau du collagène. Des protéines appelées métalloprotéases (MMPs) permettent de contrôler la quantité de collagène déposée de manière anarchique dans les phases précoces du processus tandis que la synthèse de collagène persiste pour reconstituer des fibres régulières et bien ordonnées. Les inhibiteurs de ces MMPs régulent l'activité de ces enzymes. Un équilibre se crée donc entre la formation du nouveau collagène et la destruction de l'ancien collagène. Le collagène III initialement déposé diminue fortement pour laisser la place au collagène I.

Avec l'âge, au cours du vieillissement cutané, la réparation tissulaire devient moins efficace : en particulier, les fibroblastes prolifèrent et migrent moins, la synthèse de collagène diminue.

Les demanderesses ont mis au point un procédé permettant l'obtention d'un mélange d'oligosaccharides neutres issus des graines de lin (notamment *Linum usitatissimum),* ledit mélange obtenu par le procédé selon l'invention permettant, par sa composition particulière en oligosaccharides, une amélioration substantielle de l'efficacité des processus mis en place lors de la réparation tissulaire tout en favorisant un ralentissement de l'apparition des signes visibles du vieillissement cutané.

Les expériences et recherches qui ont été menées mettent en évidence que le mélange d'oligosaccharides présentant des masses molaires issues d'un fractionnement par ultrafiltration sur seuil de coupure compris d'une part entre 5 000 et 15 000 Da et d'autre part entre 15 000 et 50 000 Da, et obtenu par le procédé selon l'invention, est particulièrement intéressant pour améliorer la réparation cutanée.

Avantageusement, un tel mélange comprenant préférentiellement une pluralité d'oligosaccharides est obtenu, par la mise en oeuvre du procédé selon l'invention, à partir d'une solution de mucilage de graines de lin et présente des propriétés remarquables, permettant notamment une action positive sur les cellules intervenant dans les mécanismes de la réparation tissulaire, telles que les fibroblastes et les kératinocytes.

Selon un mode de réalisation préférentiel, le mélange d'oligosaccharides neutres, issus de graines de lin par la mise en oeuvre du procédé selon l'invention, peut être utilisé pour favoriser la cicatrisation d'une plaie, aussi bien chez l'Homme que chez l'animal.

Ainsi, avantageusement, un tel mélange peut être intégré à un dispositif médical de type patch ou pansement, ce dernier étant destiné à être appliqué au niveau d'une plaie quelconque de sorte à permettre une cicatrisation plus rapide de cette dernière.

De façon particulièrement intéressante, le mélange d'oligosaccharides obtenu par le présent procédé peut également être utilisé en tant que médicament, que ce soit dans le domaine de la médecine humaine ou de la médecine vétérinaire.

En particulier, ledit mélange d'oligosaccharides peut avantageusement favoriser une cicatrisation des tissus, notamment dans les ulcères chroniques ou suite à une intervention chirurgicale, en particulier par son action bénéfique sur les fibroblastes des tissus conjonctifs.

Les propriétés du mélange d'oligosaccharides neutres issus des graines de lin, obtenu par le procédé selon l'invention, seront développées et illustrées dans les exemples ci-dessous, en relation avec les différentes figures annexées.

### Exemple 1 : Procédé de préparation des fractions oligosaccharidiques

Dans le procédé selon l'invention, une solution de mucilage est obtenue par extraction de graines de lin jaune, dans un solvant et préférentiellement à chaud, par exemple à une température de l'ordre de 80°C. Lesdites graines de lin proviennent notamment de *Linum usitatissimum* et le ratio entre lesdites graines et le solvant, avantageusement de l'eau, est préférentiellement de l'ordre de 1/10.

Cette étape d'extraction est avantageusement suivie d'une séparation centrifuge, et un extrait liquide est récupéré et précipité en milieu éthanolique, en mettant en oeuvre un ratio de l'ordre de 1/4 entre ledit extrait et l'éthanol.

Avantageusement, l'extrait est ensuite remis en solution à 2% pour l'étape d'hydrolyse. Cette solution est hydrolysée à pH 2, préférentiellement par ajout d'acide sulfurique H₂SO₄. La réaction d'hydrolyse est effectuée à une température de l'ordre de 80°C, pendant une durée de 24h.

Suite à l'étape d'hydrolyse, la solution est neutralisée, par exemple par ajout d'une quantité adéquate d'une base forte choisie dans le groupe comprenant notamment l'hydroxyde de baryum Ba(OH)₂ et l'hydroxyde de sodium NaOH.

Cependant, il est également possible d'utiliser toute autre base permettant une neutralisation de la solution de mucilage.

La solution subit ensuite une série de plusieurs ultrafiltrations pour être purifiée.

La première ultrafiltration de la solution est effectuée au travers d'une membrane préférentiellement minérale ayant un seuil de porosité de 50 000 Da (Carbosep, diamètre 6mm, trilobée, surface 6,8m²). Suite à cette première ultrafiltration, réalisée de préférence avec un facteur de concentration volumique entre 5 et 7, on obtient un premier rétentat, comportant les composés et molécules ayant des masses molaires ne passant pas le seuil de coupure de 50 000 Da, et un premier perméat, dans lequel on retrouve les composés de masses molaires passant ce seuil de porosité.

Ce premier perméat subit alors une seconde étape d'ultrafiltration, (de préférence sur membrane minérale 15 000 Da, InsideCéram, diamètre 20mm / 13 canaux, surface de 0,8m²) réalisée avantageusement avec un facteur de concentration volumique entre 4 et 8, et l'on obtient un second rétentat et un second perméat.

Le second perméat, obtenu suite à la seconde étape d'ultrafiltration sur membrane 15 000 Da, subit encore une étape d'ultrafiltration (de préférence sur membrane minérale 5 000 Da, InsideCéram, 20mm / 13 canaux, surface de 0,8m²) réalisée par exemple avec un facteur de concentration volumique compris entre 4 et 8. L'on obtient alors un troisième rétentat et un troisième perméat.

A la fin des ultrafiltrations, 7 échantillons, ou fractions, sont donc obtenus : l'hydrolysat de départ (E1), le premier rétentat 50 000 Da (E2), le premier perméat 50 000 Da (E3), le second rétentat 15 000 Da (E4), le second perméat 15 000 Da (E5), le troisième rétentat 5 000 Da (E6) et le troisième perméat 5 000 Da (E7).

Plus particulièrement, les échantillons d'intérêt sont les échantillons référencés E4 (second rétentat) et E6 (troisième rétentat), comprenant respectivement des oligosaccharides ayant des masses molaires élevées issues du fractionnement par ultrafiltration sur seuil de coupure compris d'une part entre 15 000 et 50 000 Da pour E4 et d'autre part entre 5 000 et 15 000 Da pour E6.

Ces échantillons sont laissés sous forme liquide ou lyophilisés.

Le mélange des second et troisième rétentats permet d'obtenir le mélange d'oligosaccharides d'intérêt.

### Exemple 2 : Caractérisation des fractions oligosaccharidiques

Les échantillons ont été analysés par chromatographie d'exclusion stérique (SEC: Size Exclusion Chromatography) couplée à la diffusion de la lumière (MALS : Multi Angle Light Scattering) et d'un détecteur réfractométrique différentiel (DRI: Differential Refractive Index). La ligne de l'analyse est composée d'un dégazeur (DGU-20A3 Shimadzu, Japon), d'une pompe HPLC (LC10Ai Shimadzu, Japon) à un débit de 0,5 mL/min, d'un injecteur automatique (SIL-20A Shimadzu, Japon), de deux colonnes Shodex montées en série (OHpack SB802,5 et SB804), d'un détecteur de diffusion de la lumière multiangles : MALS (Dawn EOS, Wyatt Technology Corp., USA), équipé d'une cellule K5 de 50µL et de 18 diodes de mesure et d'un détecteur réfractométrique différentiel (RID 10A Shimadzu, Japon). Les fractions sont caractérisées à l'aide du logiciel Astra 6 (Wyatt Technology), en utilisant la méthode Zimm ordre 1. L'incrément d'indice de réfraction (dn/dc) utilisé est de 0,15 mL/g, valeur moyenne classique pour un oligosaccharide ou un polysaccharide.

Les solutions avec des concentrations à 2, 5, 10 ou 20 g/L d'oligosaccharides, selon les fractions, sont réalisées soit par solubilisation dans une solution de NaCl 0,15 mol/L du mélange d'oligosaccharides lyophilisé, soit par dilution du mélange sous forme liquide en tenant compte de l'extrait sec de la solution initiale. Un volume de 30 mL de ces solutions est prélevé et filtré sous vide pendant 10 minutes à l'aide d'une membrane de cellulose régénérée de porosité 0,45pm de marque Millipore pour éliminer tout composé insoluble surdiffusant et pour obtenir une belle ligne de base.

L'intégration des pics réfractométrique et de diffusion de la lumière donne, pour le second rétentat (E4), issu du fractionnement par ultrafiltration sur seuil de coupure entre 15 000 et 50 000 Da, une masse molaire moyenne en nombre de 17 000 g/mol et une masse molaire moyenne en poids de 31 000 g/mol (incertitudes de mesures +/- 2 000 g/mol).

Pour le troisième rétentat (E6), issu des seuils de coupure 5 000 et 15 000 g/mol, les masses molaires moyennes en nombre et en poids sont respectivement de 4 000 et 5 000 g/mol avec une incertitude de mesure de +/- 1 000 g/mol.

Les incertitudes de mesure sont dues au faible signal de diffusion de la lumière en raison des masses molaires faibles.

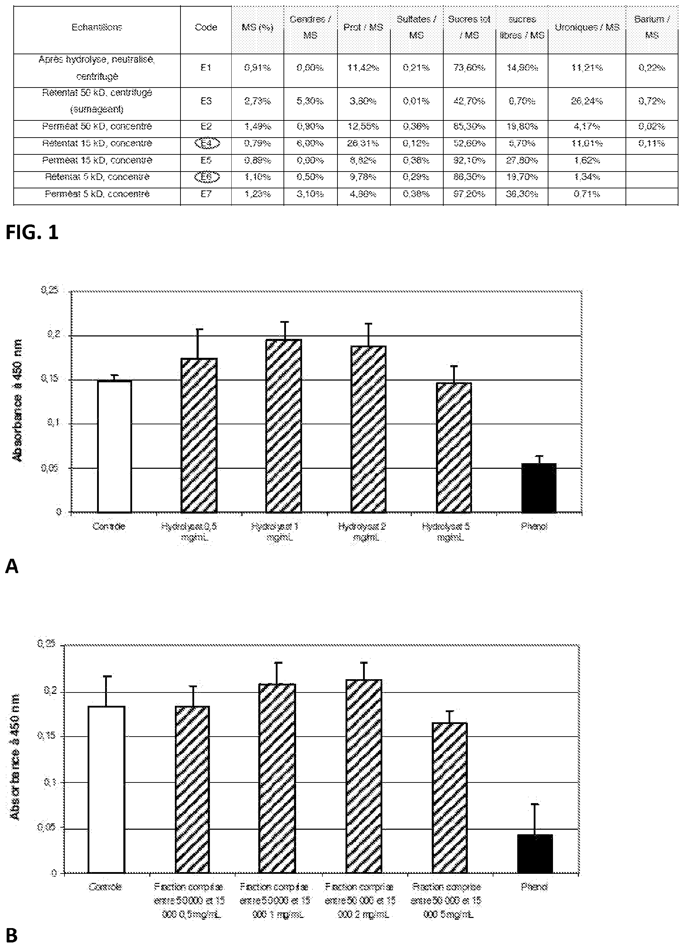

Les autres résultats obtenus sont listés dans le tableau de la figure 1.

Dans ce tableau, MS correspond à la masse sèche récupérée. Le terme « cendres/MS » correspond à la teneur en cendres par rapport à la matière sèche de l'échantillon.

La teneur en matière sèche est déterminée par séchage à l'étuve dans les conditions suivantes 16 heures minimum à 103°C.

La teneur en cendres est déterminée par traitement de l'échantillon dans un four à moufles dans les conditions suivantes : gradient de température (103°C, 550°C, 700°C) pendant 20h.

Le terme « Prot » est relatif au pourcentage de protéines dosées par la méthode de Kjeldahl.

La colonne « Uroniques » permet d'illustrer la quantité d'acides uroniques récupérés, dosés par colorimétrie.

Le pourcentage d'oligosaccharides de chacun des échantillons peut être déterminé en soustrayant le pourcentage de sucres libres du pourcentage de sucres total.

Il est remarquable que le pourcentage d'acides uroniques dans les échantillons analysés est faible, préférentiellement inférieur à 12%, et plus préférentiellement encore inférieur à 5%, et ce notamment dans les fractions d'intérêt E4 et E6.

De plus, après analyse des profils oligosaccharidiques dans les différents échantillons (résultats non montrés), il s'avère que, dans les second et troisième rétentats, comportant le mélange d'oligosaccharides d'intérêt, les chaines oligosaccharidiques comportent peu de rhamnose.

En effet, les chaines osidiques ionisées comportant du rhamnose et des acides uroniques sont peu affectées par l'hydrolyse, c'est pourquoi le mélange d'oligosaccharides comporte peu de ces molécules.

Ainsi, avantageusement, la fraction neutre des oligosaccharides, qui constitue la fraction la plus active, est la plus affectée par l'étape d'hydrolyse du procédé selon l'invention.

Le profil des oligosaccharides a également été analysé dans les échantillons. Il a notamment été trouvé que le pourcentage d'oligosaccharides dans chacun des échantillons est compris entre 40 et 80% en masse par rapport au poids total dudit échantillon.

Par ailleurs, il a été mis en évidence que les chaînes des oligosaccharides présents dans les échantillons comprenaient notamment les oses suivants : fucose, arabinose, galactose, glucose et xylose.

Effets des fractions oligosaccharidiques sur les cellules cutanées

Les différents échantillons E1 à E7 ont été testés pour évaluer leurs effets, en particulier sur les cellules intervenant notamment dans les processus de réparation cutanée.

### Exemple 3 : Absence de cytotoxicité des fractions obtenues sur les fibroblastes et sur les kératinocytes

Des fibroblastes dermiques et des kératinocytes épidermiques provenant de peaux adultes (fragments de peaux obtenus lors d'interventions chirurgicales ou cultures cellulaires commerciales (Lonza®, Suisse)) ont été mis en culture *in vitro.* Les expériences ont été conduites sur des cellules en cultures primaires pour se rapprocher au mieux des conditions existant *in vivo.*

Les études menées sur ces cultures cellulaires ont permis de mettre en évidence que, jusqu'à une concentration de l'ordre de 5mg/mL d'oligosaccharides, les échantillons comportant des oligosaccharides issus de graines de lin ne sont pas cytotoxiques pour les cellules de type fibroblastes dermiques ou kératinocytes épidermiques.

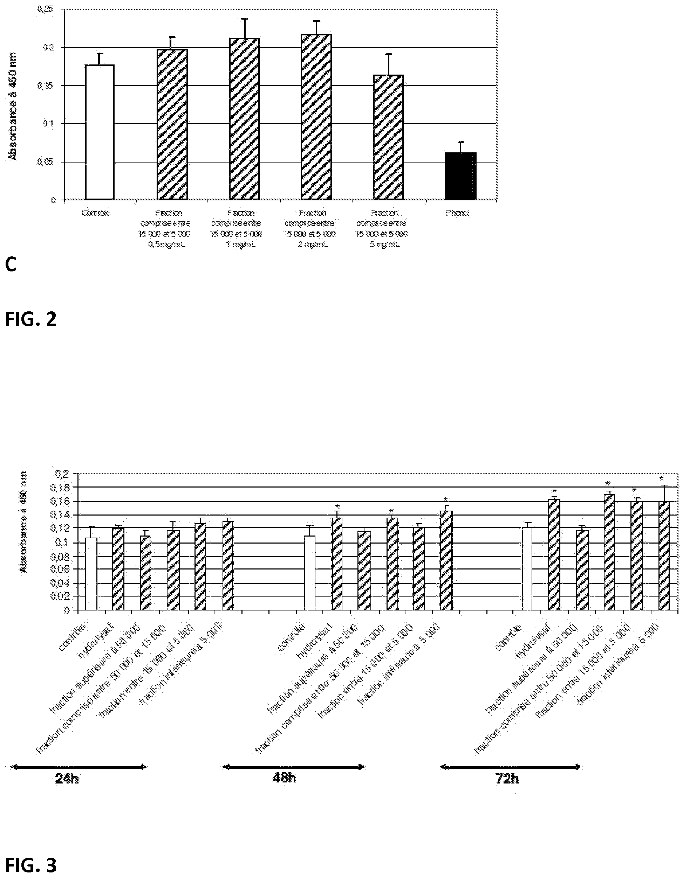

Les résultats obtenus sont visibles sur les histogrammes de la figure 2 ci-jointe. Celle-ci illustre le nombre de cellules vivantes, représenté par l'absorbance à 450nm en ordonnées, lorsque ces cellules sont en présence des différentes fractions oligosaccharidiques.

Plus particulièrement, les fractions qui ont été testées sont l'hydrolysat E1 (figure 2A), le second rétentat E4 (figure 2B - fraction comprenant les oligosaccharides dont les masses molaires sont comprises entre les seuils de coupures d'ultrafiltration de 15 000 et 50 000 Da) et le rétentat E6 (figure 2C - fraction comprenant les oligosaccharides dont les masses molaires sont comprises entre les seuils de coupures d'ultrafiltration de 5 000 et 15 000 Da).

Ces fractions ont été testées à différentes concentrations (0,5 ; 1,0 ; 2,0 et 5,0mg/mL) sur des fibroblastes dermiques humains adultes cultivés *in vitro* et arrivés à confluence. Le contrôle correspond à la cytotoxicité du milieu de culture seul et le témoin cytotoxique correspond à des cellules mises en culture dans du phénol (0,1%).

Pour évaluer la cytotoxicité des différents échantillons sur les cellules le test WST-1 est utilisé.

Les résultats montrent que les échantillons qui ont été testés ne sont pas cytotoxiques pour les cellules, et n'entraînent pas de dommages, d'une part sur les fibroblastes et d'autre part sur les kératinocytes (résultats non montrés) jusqu'à une concentration en oligosaccharides de 5mg/mL.

De plus, d'autres expériences menées ont montré qu'il n'y avait pas d'effets délétères suite à l'exposition de fibroblastes et de kératinocytes à des fractions oligosaccharidiques présentant une concentration de 1mg/mL pendant une durée de 48h (résultats non montrés).

### Exemple 4 : Effet des fractions oligosaccharidiques sur la prolifération des fibroblastes

Il a été démontré que, en plus de ne pas avoir d'effets cytotoxiques sur les cellules, les oligosaccharides issus de graines de lin, et obtenus selon le procédé de l'invention, permettent une stimulation efficace de la prolifération des fibroblastes dermiques.

L'effet positif des oligosaccharides neutres de graines de lin sur les fibroblastes est illustré sur la figure 3. Les cellules ont été exposées à des fractions oligosaccharidiques présentant une concentration de 1mg/mL, et la prolifération des fibroblastes est mesurée par la mise en oeuvre du test WST-1 à différents temps d'exposition (24, 48 et 72h).

Les résultats montrent que les fractions oligosaccharidiques qui ont été testées induisent une augmentation de la prolifération des fibroblastes dermiques. La stimulation de la prolifération est d'autant plus marquée après 48h ou 72h d'incubation des cellules en présence des oligosaccharides issus des graines de lin.

Des études sur la prolifération des fibroblastes en fonction de la concentration des fractions oligosaccharides ont également été conduites. Les résultats (non montrés) permettent d'observer que la stimulation de la prolifération est observée dès 0,5mg/mL pour les échantillons E4 et E6.

### Exemple 5 : Effet des oligosaccharides neutres de graines de lin sur le chimiotactisme

Il a encore été démontré que les oligosaccharides neutres issus des graines de lin obtenus par le procédé de l'invention présentent une importante activité chimiotactique sur les fibroblastes. Cet effet est particulièrement avantageux pour la réparation tissulaire.

Pour cela, des fibroblastes dermiques humains adultes ont été cultivés *in vitro* dans des inserts Transwell® comprenant une membrane, lesdits fibroblastes étant incubés pendant 24h en présence ou en absence (contrôle) de différents échantillons comprenant des oligosaccharides issus de graines de lin à une concentration de 1mg/mL.

Le chimiotactisme des fibroblastes dermiques est évalué par un comptage du nombre de cellules qui traversent la membrane de l'insert.

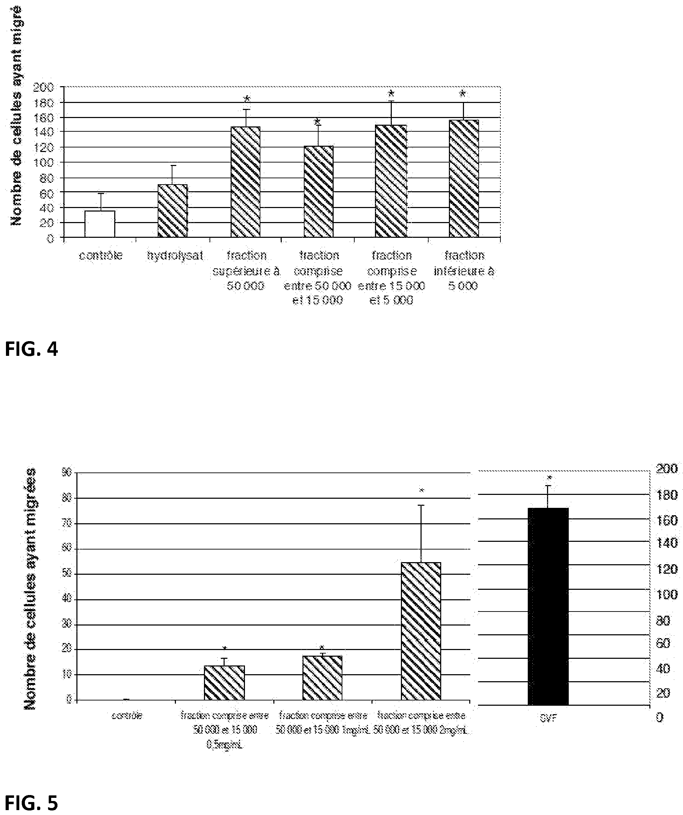

Les résultats, visibles sur la figure 4, montrent clairement que les oligosaccharides neutres de lin, obtenus par le procédé selon l'invention, induisent une augmentation du chimiotactisme des fibroblastes dermiques. D'autre expériences menées ont permis de montrer que l'effet chimiotactique est visible à une concentration très faible en oligosaccharides (0,5mg/mL), cet effet chimiotactique augmentant avec des concentrations plus élevées en oligosaccharides. Les résultats sont illustrés sur la figure 5.

### Exemple 6 : Effet des oligosaccharides de graines de lin sur la migration cellulaire

La migration cellulaire des fibroblastes dermiques au cours du temps a été mesurée en présence et en absence d'oligosaccharides neutres provenant de graines de lin et obtenus par le procédé selon l'invention. Ces composés ont été testés sur des fibroblastes et la migration de ces derniers a été suivie au cours du temps.

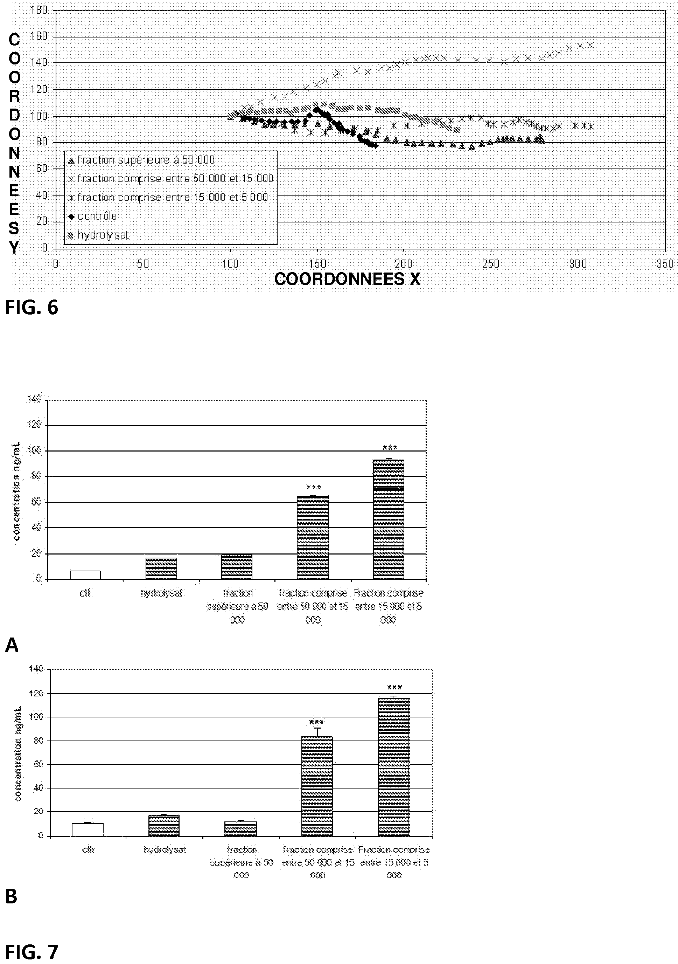

Les résultats sont illustrés sur la figure 6. Des fibroblastes dermiques humains adultes ont été cultivés *in vitro* dans des inserts Ibidi® permettant de mettre en place les conditions d'une blessure artificielle.

Les fibroblastes ont été incubés en présence d'une concentration en oligosaccharides de 1mg/mL et la migration des cellules est suivie pendant 48h au moyen d'un vidéomicroscope. La trajectoire des cellules est ainsi reconstituée.

Il est nettement visible que les fractions oligosaccharidiques, obtenues par la mise en oeuvre du procédé selon l'invention et présentant des oligosaccharides neutres ayant des masses molaires issues du fractionnement par Ultrafiltration sur seuil de coupure entre 5000 et 15 000 Da et entre 15 000 et 50 000 Da, permettent une migration cellulaire importante, ce qui induit une fermeture beaucoup plus rapide d'une blessure artificielle *in vitro.*

### Exemple 7 : Effet des oligosaccharides de graines de lin sur la synthèse de collagène de type III et de type IV

Il a été démontré ici que les fractions comportant des oligosaccharides neutres issus de la graine de lin entrainent une augmentation significative de la synthèse de collagène, et surtout du collagène de type III qui est synthétisé en premier lieu lors de la réparation tissulaire par les fibroblastes.

Des fibroblastes dermiques humains adultes ont été mis en culture *in vitro* et ont été activés pendant 24h (figure 7A) et 48h (figure 7B) en présence et en absence de fractions oligosaccharidiques issues de graines de lin, celles-ci présentant une concentration de 1mg/mL. Les surnageants de culture ont été récupérés, et la quantité de collagène de type III a été déterminée par un dosage à l'aide d'un kit commercial ELISA.

Les résultats de la synthèse de collagène de type III sont visibles sur la figure 7.

Cette figure illustre bien que les échantillons comportant d'une part des oligosaccharides neutres ayant des masses molaires issues du fractionnement par ultrafiltration sur seuil de coupure entre 5 000 et 15 000 Da et d'autre part entre 15 000 et 50 000 Da, lesdits échantillons étant obtenus par le procédé selon l'invention, permettent une augmentation significative de la synthèse de collagène de type III par les fibroblastes.

D'autres expériences menées ont également permis de mettre en évidence que les oligosaccharides neutres issus de graines de lin permettent une augmentation de la synthèse de collagène de type IV par les fibroblastes.

Plus précisément, des fibroblastes dermiques humains ont été cultivés *in vitro* jusqu'à confluence, puis les cellules ont été incubées pendant 24h avec divers échantillons, notamment avec les fractions d'oligosaccharides correspondant aux second et troisième perméats et obtenues par la mise en oeuvre du procédé selon l'invention. La concentration des fractions est de 1mg/mL.

Les surnageants de culture sont ensuite récupérés, et le collagène IV est analysé dans le surnageant et dans la couche cellulaire.

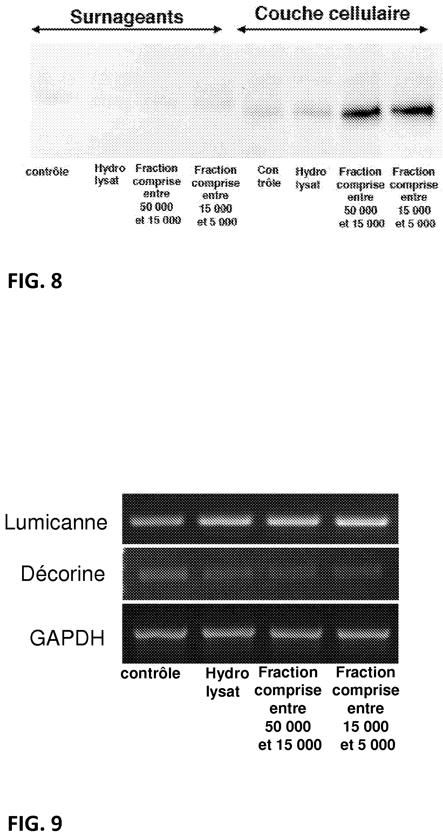

Les résultats, illustrés sur la figure 8, montrent que les fractions oligosaccharidiques, issues d'un fractionnement par ultrafiltration sur seuil de coupure compris d'une part entre 15 000 et 50 000 Da et d'autre part entre 5 000 et 15 00 Da, sont aptes à activer la synthèse de collagène IV. Un tel effet est particulièrement avantageux, car le collagène IV constitue l'un des composants essentiels des membranes basales et que la synthèse de ce composant a tendance à diminuer lors du vieillissement.

### Exemple 8 : Effets des oligosaccharides neutres issus de graines de lin sur la synthèse des petits protéoglycannes

Les composés appelés protéoglycannes, et notamment les SLRP (Small Leucine-Rich Proteoglycans), jouent un rôle important dans la régulation de l'activité cellulaire et dans l'organisation des propriétés fonctionnelles de la peau.

En particulier, la décorine et le lumicanne sont des protéoglycannes importants. La décorine est abondante dans les peaux adultes et joue un rôle primordial dans la régulation de l'homéostasie. La quantité de décorine dans la peau augmente avec l'âge. Au contraire, la quantité de lumicane a tendance à diminuer avec l'âge. Le lumicane joue un rôle dans la préservation des propriétés fonctionnelles de la peau.

Des expériences ont été menées pour déterminer l'impact des oligosaccharides neutres issus de graines de lin, et obtenus par le présent procédé, sur la synthèse de ces petits protéoglycannes.

Les fibroblastes ont été cultivés pendant 24h en présence d'oligosaccharides neutres issus de lin à une concentration de 1mg/mL.

Les ARNs ont ensuite été extraits des fibroblastes et on a procédé à une série de RT-PCRs pour évaluer l'expression des gènes codant pour la synthèse de la décorine et du lumicane. L'expression du gène de la GAPDH sert de témoin positif.

Les résultats sont illustrés sur la figure 9 annexée. Ils montrent que les oligosaccharides neutres issus de lin permettent d'induire une diminution de la synthèse de décorine par les fibroblastes dermiques tandis que, au contraire, l'expression du lumicane augmente.

En conséquence, la mise en contact d'une peau âgée avec des oligosaccharides neutres, obtenus par le présent procédé et ayant notamment des masses molaires issues du fractionnement par ultrafiltration sur seuil de coupure entre 5 000 et 15 000 Da ou entre 15 000 et 50 000 Da, permet de se rapprocher du phénotype d'une peau plus jeune.

### Exemple 9 : Effets des oligosaccharides neutres issus de graines de lin sur les kératinocytes

Il a été observé, par microscopie optique, que les fractions comportant des oligosaccharides neutres issus de graines de lin ont un effet positif sur les kératinocytes épidermiques.

En particulier, les oligosaccharides neutres induisent une différenciation des kératinocytes, celle-ci étant plus particulièrement caractérisée par un changement de morphologie.

Les observations au microscope ont été confirmées par des expériences d'immunocytochimie, dans lesquelles différents marqueurs de différenciation ont été utilisés, tels que l'involucrine, marqueur de la couche granuleuse, ou encore la filagrine et la loricrine, marqueurs de la couche cornée de l'épiderme (résultats non montrés). L'apparition de ces marqueurs dans les kératinocytes est fortement stimulée par les oligosaccharides neutres obtenus par le procédé selon l'invention.

La stimulation de la différenciation des kératinocytes est bénéfique pour la peau car elle augmente sa protection contre les agressions extérieures.

Bien entendu, l'invention n'est pas limitée aux exemples illustrés et décrits précédemment qui peuvent présenter des variantes et modifications sans pour autant sortir du cadre de l'invention.

## Revendications

1. Procédé d'obtention d'un mélange d'oligosaccharides neutres extraits de graines de lin, lesdits oligosaccharides présentant des masses molaires élevées, ledit procédé étant **caractérisé en ce qu'**il comporte les étapes suivantes :
- on effectue une hydrolyse à pH acide d'une solution de mucilage de lin, cette dernière étant obtenue par extraction de graines de lin dans un solvant, ladite hydrolyse étant effectuée à un pH de 2, à une température de l'ordre de 80°C, pendant une durée de 24h ;
- on neutralise ladite solution par ajout d'une base en quantité adéquate ;
- on effectue une première ultrafiltration de la solution au travers d'une membrane de porosité 50 000 Da, de sorte à obtenir un premier rétentat et un premier perméat ;
- on effectue une seconde ultrafiltration dudit premier perméat au travers d'une membrane de porosité 15 000 Da, de sorte à obtenir un second rétentat et un second perméat ;
- on effectue une troisième ultrafiltration dudit second perméat au travers d'une membrane de porosité 5 000 Da de sorte à obtenir un troisième rétentat et un troisième perméat ;
- on mélange le second rétentat et le troisième rétentat pour obtenir ledit mélange d'oligosaccharides, ledit second rétentat comportant des oligosaccharides ayant des masses molaires issues du fractionnement par ultrafiltration sur seuil de coupure compris entre 15 000 et 50 000 Da, et ledit troisième rétentat comportant les oligosaccharides ayant des masses molaires issues du fractionnement par ultrafiltration sur seuil de coupure entre 5 000 et 15 000 Da.

2. Procédé selon la revendication précédente **caractérisé en ce que** l'extraction de graines de lin est réalisée dans un solvant aqueux.

3. Procédé selon l'une quelconque des revendications précédentes **caractérisé en ce que** l'on neutralise ladite solution de mucilage par ajout, en quantité adéquate, d'une base forte choisie dans le groupe comprenant au moins l'hydroxyde de baryum et l'hydroxyde de sodium.

4. Mélange d'oligosaccharides neutres extraits de graines de lin obtenu par la mise en oeuvre du procédé selon l'une quelconque des revendications précédentes, **caractérisé en ce que** ledit mélange comporte des oligosaccharides ayant des masses molaires issues d'un fractionnement par ultrafiltration sur seuil de coupure compris d'une part entre 15 000 et 50 000 Da et d'autre part entre 5 000 et 15 000 Da.

5. Mélange d'oligosaccharides neutres extraits de graines de lin selon la revendication 4 **caractérisé en ce qu'**il comporte des oligosaccharides dont la chaîne comprend au moins du fucose et/ou de l'arabinose et/ou du galactose et/ou du glucose et/ou du xylose.

6. Mélange d'oligosaccharides neutres extraits de graines de lin selon les revendications 4 ou 5 **caractérisé en ce que** ledit mélange comporte un faible taux d'oligosaccharides dont la chaîne comprend du rhamnose et un faible taux d'acides uroniques.

7. Utilisation d'un mélange d'oligosaccharides neutres extraits de graines de lin selon l'une des revendications précédentes pour lutter contre les effets du vieillissement cutané.

8. Utilisation d'un mélange d'oligosaccharides neutres extraits de graines de lin selon la revendication 7 pour stimuler la prolifération des fibroblastes.

9. Utilisation d'un mélange d'oligosaccharides neutres extraits de graines de lin selon la revendication 7 pour stimuler le chimiotactisme des fibroblastes.

10. Utilisation d'un mélange d'oligosaccharides neutres extraits de graines de lin selon la revendication 7 pour stimuler la migration cellulaire des fibroblastes.

11. Utilisation d'un mélange d'oligosaccharides neutres extraits de graines de lin selon la revendication 7 pour stimuler la synthèse de collagène de type III et/ou de type IV par les fibroblastes.

12. Utilisation d'un mélange d'oligosaccharides neutres extraits de graines de lin selon la revendication 7 pour stimuler la synthèse de lumicane et pour inhiber la synthèse de décorine par les fibroblastes.

13. Utilisation d'un mélange d'oligosaccharides neutres extraits de graines de lin selon la revendication 7 pour induire la différenciation des kératinocytes.

14. Composition dermatologique ou cosmétique comprenant un mélange d'oligosaccharides neutres selon l'une quelconque des revendications 1 à 6 et au moins un véhicule cosmétiquement ou dermatologiquement acceptable.

15. Composition dermatologique ou cosmétique selon la revendication précédente dans laquelle la concentration en oligosaccharides neutres est comprise entre 0,1 et 5mg/mL.

16. Composition dermatologique ou cosmétique selon les revendications 14 ou 15 **caractérisée en ce qu'**elle se présente sous la forme d'une crème, d'un gel, d'une lotion, d'un sérum, d'une mousse ou d'une pommade.

17. Composition dermatologique ou cosmétique selon l'une des revendication 14 à 16 destinée à être appliquée sur la peau ou au niveau des phanères.

18. Mélange d'oligosaccharides neutres extraits de graines de lin selon l'une des revendications 1 à 6 pour son utilisation comme médicament.

19. Mélange d'oligosaccharides neutres extraits de graines de lin pour son utilisation selon la revendication précédente pour favoriser la cicatrisation des tissus, notamment dans les ulcères chroniques ou après une intervention chirurgicale.

20. Mélange d'oligosaccharides neutres extraits de graines de lin pour son utilisation selon la revendication 18 pour favoriser la réparation tissulaire cutanée, notamment la cicatrisation d'une plaie.

## Patentansprüche

1. Verfahren zum Erhalten einer Mischung von aus Leinsamen extrahierten neutralen Oligosacchariden, wobei die besagten Oligosaccharide hohe Molekulargewichte aufweisen, wobei das Verfahren **dadurch gekennzeichnet ist, dass** es die folgenden Schritte umfasst:
- eine Hydrolyse wird bei einem sauren pH-Wert mit einer Leinschleimlösung durchgeführt, wobei letztere durch Extraktion von Leinsamen in einem Lösungsmittel erhalten wird, wobei die besagte Hydrolyse bei einem pH-Wert von 2, bei einer Temperatur von ungefähr 80°C während einem Zeitraum von 24 Stunden durchgeführt wird ;
- die besagte Lösung wird durch Zugabe einer Base in ausreichender Menge neutralisiert;
- es wird eine erste Ultrafiltration der Lösung durch eine Membran mit einer Porosität von 50 000 Da durchgeführt, sodass ein erstes Retentat und ein erstes Permeat erhalten werden ;
- es wird eine zweite Ultrafiltration des besagten ersten Retentats durch eine Membran mit einer Porosität von 15 000 Da durchgeführt, sodass ein zweites Retentat und ein zweites Permeat erhalten werden ;
- es wird eine dritte Ultrafiltration des besagten zweiten Permeats durch eine Membran mit einer Porosität von 5 000 Da durchgeführt, sodass ein drittes Retentat und ein drittes Permeat erhalten werden ;
- das zweite Retentat und das dritte Retentat werden gemischt, um die besagte Mischung von Oligosacchariden zu erhalten, wobei das besagte zweite Retentat Oligosaccharide umfasst, die sich aus der Fraktionierung durch Ultrafiltration auf eine Schnittschwelle zwischen 15 000 und 50 000 Da ergebende Molekulargewichten hat, und wobei das besagte dritte Retentat Oligosaccharide umfasst, die sich aus der Fraktionierung durch Ultrafiltration auf eine Schnittschwelle zwischen 5 000 und 15 000 Da ergebende Molekulargewichten hat.

2. Verfahren nach dem vorhergehenden Anspruch, **dadurch gekennzeichnet, dass** die Extraktion von Leinsamen in einem wässrigen Lösungsmittel durchgeführt wird.

3. Verfahren nach irgendeinem der vorhergehenden Ansprüche, **dadurch gekennzeichnet, dass** die besagte Schleimlösung durch Zugabe in geeigneter Menge einer starken Base neutralisiert wird, die aus der Gruppe, die mindestens Bariumhydroxid und Natriumhydroxid umfasst, ausgewählt wird.

4. Mischung von aus Leinsamen extrahierten neutralen Oligosacchariden, die durch Durchführung des Verfahrens nach irgendeinem der vorhergehenden Ansprüche erhalten wird, **dadurch gekennzeichnet, dass** die besagte Mischung Oligosaccharide umfasst, die sich aus der Fraktionierung durch Ultrafiltration auf eine Schnittschwelle zwischen einerseits 15 000 und 50 000 Da und andererseits 5 000 und 15 000 Da ergebende Molekulargewichten hat.

5. Mischung von aus Leinsamen extrahierten neutralen Oligosacchariden nach Anspruch 4, **dadurch gekennzeichnet, dass** sie Oligosaccharide umfasst, deren Kette mindestens Fucose und/oder Arabinose und/oder Galactose und/oder Glucose und/oder Xylose umfasst.

6. Mischung von aus Leinsamen extrahierten neutralen Oligosacchariden nach den Ansprüchen 4 oder 5, **dadurch gekennzeichnet, dass** die besagte Mischung eine niedrige Rate von Oligosacchariden umfasst, deren Kette Rhamnose und eine niedrige Rate von Uronsäuren umfasst.

7. Verwendung einer Mischung von aus Leinsamen extrahierten neutralen Oligosacchariden nach einem der vorhergehenden Ansprüche zur Bekämpfung der Auswirkungen der Hautalterung.

8. Verwendung einer Mischung von aus Leinsamen extrahierten neutralen Oligosacchariden nach Anspruch 7 zur Stimulierung der Proliferation der Fibroblasten.

9. Verwendung einer Mischung von aus Leinsamen extrahierten neutralen Oligosacchariden nach Anspruch 7 zur Stimulierung der Chemotaxis der Fibroblasten.

10. Verwendung einer Mischung von aus Leinsamen extrahierten neutralen Oligosacchariden nach Anspruch 7 zur Stimulierung der Zellwanderung der Fibroblasten.

11. Verwendung einer Mischung von aus Leinsamen extrahierten neutralen Oligosacchariden nach Anspruch 7 zur Stimulierung der Synthese von Typ III- und/oder Typ IV-Kollagen durch die Fibroblasten.

12. Verwendung einer Mischung von aus Leinsamen extrahierten neutralen Oligosacchariden nach Anspruch 7 zur Stimulierung der Lumicansynthese und zur Inhibierung der Decorinsynthese durch die Fibroblasten.

13. Verwendung einer Mischung von aus Leinsamen extrahierten neutralen Oligosacchariden nach Anspruch 7 zur Induktion der Differenzierung der Keratinozyten.

14. Dermatologische oder kosmetische Zusammensetzung, umfassend eine Mischung von neutralen Oligosacchariden nach irgendeinem der Ansprüche 1 bis 6 und mindestens einen kosmetisch oder dermatologisch verträglichen Träger.

15. Dermatologische oder kosmetische Zusammensetzung nach dem vorhergehenden Anspruch, bei der die Konzentration an neutralen Oligosacchariden zwischen 0,1 und 5 mg/ml liegt.

16. Dermatologische oder kosmetische Zusammensetzung nach den Ansprüchen 14 oder 15, **dadurch gekennzeichnet, dass** sie in Form einer Creme, eines Gels, einer Lotion, eines Serums, eines Schaums oder einer Salbe vorliegt.

17. Dermatologische oder kosmetische Zusammensetzung nach einem der Ansprüche 14 bis 16, die zum Auftragen auf die Haut oder im Bereich der Hautanhangsgebilde bestimmt ist.

18. Mischung von aus Leinsamen extrahierten neutralen Oligosacchariden nach einem der Ansprüche 1 bis 6 für ihre Verwendung als Medikament.

19. Mischung von aus Leinsamen extrahierten neutralen Oligosacchariden für ihre Verwendung nach dem vorhergehenden Anspruch zur Förderung der Heilung der Geweben, nämlich bei chronischen Geschwüren oder nach einem chirurgischen Eingriff.

20. Mischung von aus Leinsamen extrahierten neutralen Oligosacchariden für ihre Verwendung nach Anspruch 18 zur Förderung der Reparatur des Hautgewebes, nämlich zur Heilung einer Wunde.

## Claims

1. A method for obtaining a mixture of neutral oligosaccharides extracted from flax seeds, said oligosaccharides having high molecular weights, wherein said method includes the following steps :
- an hydrolysis at acidic pH is carried out on a solution of flax mucilage, the latter being obtained by extraction from flax seeds in a solvent, said hydrolysis being carried out at a pH of 2, at a temperature of about 80°C, for a 24 hours' period ;
- said solution is neutralized by adding a base in an adequate quantity ;
- a first ultrafiltration of the solution through a membrane with a porosity of 50,000 Da is carried out, so as to obtain a first retentate and a first permeate ;
- a second ultrafiltration of said first permeate through a membrane with a porosity of 15,000 Da is carried out, so as to obtain a second retentate and a second permeate ;
- a third ultrafiltration of said second permeate through a membrane with a porosity of 5,000 Da is carried out, so as to obtain a third retentate and a third permeate;
- the second and the third retentate are mixed in order to obtain said mixture of oligosaccharides, said second retentate including oligosaccharides having molar weights resulting from the fractionation by ultrafiltration at a cutoff point between 15,000 and 50,000 Da, and said third retentate including the oligosaccharides having molar weights resulting from the fractionation by ultrafiltration at a cutoff point between 5,000 and 15,000 Da.

2. The method according to the preceding claim, wherein the extraction from flax seeds is carried out in an aqueous solvent.

3. The method according to any of the preceding claims, wherein said solution of mucilage is neutralized by adding an adequate quantity of a strong base selected among the group comprising at least barium hydroxide and sodium hydroxide.

4. A mixture of neutral oligosaccharides extracted from flax seeds obtained by implementing the method according to any of the preceding claims, wherein said mixture includes oligosaccharides having molecular weights resulting from a fractionation by ultrafiltration at a cutoff point, on the one hand, between 15,000 and 50,000 Da and, on the other hand, between 5,000 and 15,000 Da.

5. The mixture of neutral oligosaccharides extracted from flax seeds according to claim 4, wherein it includes oligosaccharides the chain of which comprises at least fucose and/or arabinose and/or galactose and/or glucose and/or xylose.

6. The mixture of neutral oligosaccharides extracted from flax seeds according to claim 4 or 5, wherein said mixture includes a low rate of oligosaccharides the chain of which comprises rhamnose and a low rate of uronic acids.

7. A use of a mixture of neutral oligosaccharides extracted from flax seeds according to one of the preceding claims for fighting the effects of skin aging.

8. The use of a mixture of neutral oligosaccharides extracted from flax seeds according to claim 7 for stimulating the proliferation of fibroblasts.

9. The use of a mixture of neutral oligosaccharides extracted from flax seeds according to claim 7 for stimulating the chemotaxis of fibroblasts.

10. The use of a mixture of neutral oligosaccharides extracted from flax seeds according to claim 7 for stimulating the cell migration of fibroblasts.

11. The use of a mixture of neutral oligosaccharides extracted from flax seeds according to claim 7 for stimulating the synthesis of type III and/or type IV collagen by the fibroblasts.

12. The use of a mixture of neutral oligosaccharides extracted from flax seeds according to claim 7 for stimulating the lumicane synthesis and for inhibiting the decorin synthesis by the fibroblasts.

13. The use of a mixture of neutral oligosaccharides extracted from flax seeds according to claim 7 for inducing the differentiation of the keratinocytes.

14. A dermatological or cosmetic composition comprising a mixture of neutral oligosaccharides according to any one of claims 1 to 6 and at least one cosmetically or dermatologically acceptable vehicle.

15. The dermatological or cosmetic composition according to the preceding claim, wherein the concentration of neutral oligosaccharides is between 0.1 and 5 mg/ml.

16. The dermatological or cosmetic composition according to claims 14 or 15, wherein it is in the form of a cream, a gel, a lotion, a serum, a foam or an ointment.

17. The dermatological or cosmetic composition according to one of claims 14 to 16 intended to be applied on the skin or at the level of the integuments.

18. The mixture of neutral oligosaccharides extracted from flax seeds according to one of claims 1 to 6 for its use as a medicament.

19. The mixture of neutral oligosaccharides extracted from flax seeds for its use according to the preceding claim for promoting the healing of the tissues, namely in the chronic ulcers or after a surgical intervention.

20. The mixture of neutral oligosaccharides extracted from flax seeds for its use according to claim 18 for promoting the skin tissue repair, namely the healing of a wound.
